# EUROPEAN PATENT APPLICATION

(11) **EP 4 653 452 A1**
(43) Date of publication of application: **26.11.2025**
(21) Application number: 24705210.3
(22) Date of filing: 03.01.2024
(51) Int. Cl.: C07K 9/00, G01N 33/543, G01N 33/574

(54) **SYNTHETIC GLYCOPEPTIDES AND DERIVATIVES FOR THE EARLY-STAGE DETECTION OF CANCER BIOMARKERS IN MUC1-POSITIVE TUMORS**

(30) Priority: 19.01.2023 ES 202330032
(71) Applicant: Fundación Rioja Salud, 26006 Logroño (La Rioja) (ES); Universidad De La Rioja, 26006 Logroño (La Rioja) (ES); Università Degli Studi Di Verona, 37129 Verona (IT)
(72) Inventor: MARTÍNEZ RAMÍREZ, Alfredo, 26006 Logroño (La Rioja) (ES); MARTÍN CARNICERO, Alfonso, 26006 Logroño (La Rioja) (ES); MARTÍNEZ MORAL, Pilar, 26006 Logroño (La Rioja) (ES); CORZANA LÓPEZ, Francisco, 26006 Logroño (La Rioja) (ES); ASÍN VICENTE, Alicia, 26006 Logroño (La Rioja) (ES); FIAMMENGO, Roberto, 26006 Logroño (La Rioja) (ES); EGUSKIZA BILBAO, Ander, Verona (IT); MALERBA, Giovanni, Verona (IT); DE TOMI, Elisa, Verona (IT)
(74) Representative: Fernandez Pou, Felipe
(86) International application number: PCT/ES2024/070001
(87) International publication number: WO 2024/153839

(57) **Abstract**

The present invention relates to a glycopeptide derived from MUC1 the sequence of which consists of SEQ ID NO 2 (CGGGGPAPGST(GaINCOEt)APPA) for use as a pancreatic cancer biomarker by binding the glycopeptide to tumour-associated anti-MUC1 antibodies, the glycopeptide being attached to a nanoparticle. Likewise, the invention provides a kit for detecting and quantifying anti-MUC1 antibodies in a biological sample isolated from an individual, wherein the kit is configured for detecting and quantifying binding between anti-MUC1 antibodies, present in the sample, and the glycopeptide of the invention by visualising and quantifying fluorescent signals.

## Description

### Technical Field

The present invention belongs to the technical field of medicine, in particular belongs to the technical field of oncology and, more particularly, relates to synthetic glycopeptides suitable for the early detection of pancreatic and other tumours expressing MUC1.

### Background of the invention

MUC1 (mucin-1) is a glycoprotein that complexes carbohydrates bound by means of an alpha-O-glycosidic bond to threonine and/or serine side chains. Mucins coat the apical surface of epithelial cells in the lungs, the stomach, the intestines, eyes and other organs. Therefore, they protect the body from infection by binding pathogens to oligosaccharides in the extracellular domain, which prevents the pathogen from reaching and infecting the cell. Overexpression and abnormal glycosylation of MUC1 are often associated with colon, breast, ovary, lung and pancreas cancers, among others. Joyce Taylor-Papadimitriou identified and characterised the most immunogenic fragment of the MUC1 glycoprotein during her work with breast and ovarian¹ tumours.

MUC1 mucin has two subunits, C-terminal MUC and N-terminal MUC.^{2,3} The latter subunit can be detached from the surface and, as a result, detected in the serum of cancer patients.^{4,5} Aberrantly glycosylated MUC1, which leads to the presentation of simple carbohydrates is known as (TA)-MUC1 (tumour-associated MUC1). In this sense, TA-MUC1 is found at very low levels in human serum from healthy people, which increase in cases of cancer^{6,7}. Moreover, for other biomarkers, levels above 40 U/ml of CA 27-29⁸ or levels above 25-30 U/ml of CA 15-3⁹ have generally been found to suggest the presence of malignant tumours. Although many examples of antigen-based cancer biomarkers can be found in the literature, most of them do not exhibit satisfactory sensitivity and specificity.^{5,10-13}

Nevertheless, patient-generated anti-TA-MUC1 antibodies could represent an attractive alternative since, in general, persist in the blood longer than the corresponding antigens and are present in the early stages of the disease. Notably, multiple studies using MUC1 as a tool for detecting and measuring these antibodies have been reported in the literature. In this context, special attention should be paid to the system developed by Wang et al. They used a peptide possessing 6 MUC1 repeat units for detecting anti-MUC1 antibodies in human serum.¹⁴ In this line of research, Gheybi and colleagues developed a chimera consisting of MUC1 and a breast cancer-associated growth factor receptor (HER2). This combination can be used for simultaneously detecting MUC1 and HER2 antibodies in human serum.¹⁵

Today, pancreatic cancer is the fourteenth most common cancer worldwide and the eighth leading cause of cancer-related death.¹⁶ In Spain, pancreatic tumour was the third leading cause of cancer deaths in 2019.¹⁷ The lethality of this disease is a consequence of the late detection of this tumour, which is generally at a very advanced stage.¹⁸ Currently, surgery, chemotherapy and radiotherapy are the most commonly used therapies to treat pancreatic cancer, although the life expectancy of patients diagnosed with this cancer is only 4 to 6 months.¹⁹

It should be noted that, in pancreatic cancer, TA-MUC1 overexpression correlates with more metastasis and worse prognosis.²⁰ In this respect, anti-MUC1 antibodies could play a key role in the diagnosis of patients with pancreatic cancer in the early stages of the disease.²¹ Most of the examples in the literature based on the detection of anti-MUC1 antibodies do not exhibit any alteration of the natural antigens.

Two blood biomarkers are currently used for the clinical management of patients with pancreatic cancer: CA19-9²² and carcinoembryonic antigen (CEA).²³ These biomarkers, however, lack sufficient sensitivity and specificity, and therefore cannot be used as diagnostic markers, although they are currently used in the follow-up of certain patients. Therefore, finding more sensitive and specific biomarkers may provide a means of detecting pancreatic cancer at an earlier stage, which therefore provides the opportunity for early intervention and a significant increase in patient survival.

In summary, the tumour-associated form of MUC1 (TA-MUC1) causes the production of antibodies in patients. These autoantibodies may constitute an attractive biomarker for early detection of the disease and could be quantified using different immunoassay systems. All systems reported so far lack diagnostic potential due to their low sensitivity and specificity. We propose the use of antigens consisting of short artificial glycopeptides bound to nanoparticles as a more efficient detection system for patient-specific autoantibodies that can be used for early clinical diagnosis in patients.

### Description of the invention

In order to solve the drawbacks of the state of the art, modifications to the structure of the antigen could be a key point to enhance the interaction between the antigen and the TA-MUC1 antibodies generated by the patient and, therefore, improve the selectivity and sensitivity of a possible detection system.^{24,25} For this purpose, it would be essential to understand the molecular interactions by which these antibodies recognise their targets.

In this sense, our research group has obtained the X-ray structure of a MUC1 glycopeptide forming a complex with the monoclonal antibody 5E5 (anti-MUC1), which provides important information on how this antibody interacts with the antigen and, therefore, paving the way for the design of non-natural antigens with improved antigen-antibody recognition characteristics.²⁶

According to a first aspect of the invention, non-natural synthetic glycopeptides are provided, as represented by [GGGGPAPGST(GaINCOEt)APPA] o [CGGGPAPGST(GaINCOEt)APPA] and their derivative glycopeptides for use as antigens in a detection system for the TA-MUC1 antibody as a cancer biomarker.

Derivatives should be understood as other molecules containing any minor chemical modification of these glycopeptide sequences and having a higher affinity than the natural glycopeptide for anti-TA-MUC1 target antibodies.

These glycopeptides were designed to be most effective in detecting the appropriate subset of TA-MUC1 antibodies that correlate with disease status, and our data show that they are useful as diagnostic reagents for detecting pancreatic cancer and, probably, for other cancers where MUC1 is aberrantly glycosylated.

The antigen consisting of a synthetic non-natural glycopeptide, or compound [GGGGPAPGST(GaINCOEt)APPA], has 6.5 times more affinity for anti-MUC1 antibodies than the natural antigen. This makes this antigen (and its derivatives) a very useful tool for detecting cancers where MUC1 is aberrantly glycosylated.

According to a second aspect of the invention, a detection system comprising a nanoparticle and an antigen consisting of a covalently bonded synthetic non-natural glycopeptide is provided, as represented by [CGGGGPAPGST(GaINCOEt)APPA] and its glycopeptide derivatives by means of minor chemical modifications.

Although ELISA assays are widely used and offer good selectivity, some problems limit its application, such as low sample concentrations or the difficulty to perform the measurement in a reproducible way. In this context, and thanks to nanotechnology, some suitably functionalised nanomaterials have been shown to be very effective in detecting different tumour markers, leading to tests with high sensitivity and selectivity.^{27,28}

The antigen-bound gold nanoparticle array [CGGGGPAPGST(GaINCOEt)APPA] aids in antigen presentation and could increase the sensitivity of the antibody detection system. This could be extrapolated to any other combination of a nanoparticle, such as micelles, dendrimers, liposomes, hybrid and compact polymeric nanoparticles, fullerenes, carbon dots, quantum dots, silica, metal nanoparticles, among others, plus a glycopeptide according to the first aspect of the invention.

Preferably, the nanoparticle is a gold nanoparticle.

Preferably, the synthetic glycopeptide antigen is [CGGGGPAPGST(GaINCOEt)APPA].

Analysis of clinical samples has shown that the use of our non-natural glycopeptide bound to gold nanoparticles (AuNPs) provides higher sensitivity and specificity (AUC=0.918) than the currently used blood biomarkers CEA and CA19-9, which therefore indicates that our system has a competitive advantage in the field of early detection of pancreatic cancer and, probably, other cancers where MUC1 is aberrantly glycosylated.

According to a third aspect of the invention, a kit is provided for performing an assay for detecting and quantifying binding between patient-produced anti-MUC1 antibodies (the biomarker) in a sample and one of the detection systems of the first or second aspect of the invention, the kit comprising a detection system according to the first or the second aspect of the invention.

The assay can be selected from the group including dot blot, Western Blot, ELISA, SPR, liquid phase, infrared or any other suitable assay for detecting antigen-antibody interactions known in the art or developed in the future.

### Brief description of the Drawings

The present invention will now be described in more detail in the following paragraphs of this specification, just by way of example, without being intended to constitute any limitation of the scope of the present invention. The following description can be better understood when read in conjunction with the accompanying drawings, in which:
Figure 1 shows the chemical formulae of the MUC1 antigens, first the natural glycopeptide 21 (Figure 1A); and then two synthetic glycopeptides (shown in Figures 1B (glycopeptide 24) and 1C (glycopeptide 33) in accordance with one embodiment of the present invention.
Figures 2A and 2B show the concentration-response curves obtained for binding glycopeptides 21 and 24, respectively, towards the 5E5 (anti-MUC1) antibody at 20 °C.
Figure 3 schematically shows the synthetic route for preparing the glycopeptide-AuNP complex: (i) AuNP passivation with alkyl-PEG600 tloles, (ii) SM(PEG)₂ coupling, (iii) glycopeptide coupling.
Figure 4 shows the dissociation constants (K_{D}) derived from experiments for the glycopeptide-AuNP conjugates analysed.
Figure 5 shows a graph depicting anti-MUC1 antibody levels in healthy volunteers (control) and pancreatic cancer patients. The box plots represent the Interquartile Range with the median as a horizontal line. ****: *p*<0.0001
Figure 6 shows a receiver operating characteristic (ROC) curve plot representing the sensitivity and specificity of anti-MUC1 antibody values in the serum of study subjects, with an area under the curve (AUC) of 0.918 (95 % Cl: 0.832-1.000), demonstrating 85 % sensitivity and 90 % specificity, with a discrimination threshold of 11.63 arbitrary fluorescence units (AFU). The published values for CEA and CA19-9 are also plotted for comparison. See also Table 4.

### Detailed description of the invention

### 1) Glycopeptide synthesis

Glycopeptides were synthesised by stepwise microwave-assisted solid-phase synthesis (MW-SPPS) on a Liberty Blue synthesiser (CEM^{®}) using the Fmoc strategy on Rink Amide MBHA resin (0.1 mmol). The non-natural amino acids were synthesised following a slight modification of the protocol described in the literature.²⁹ The O-acetyl groups of the remaining GalNAc were removed in a mixture of NH₂NH₂/MeOH (7:3). The glycopeptides were then released from the resin and all acid-sensitive protecting groups were simultaneously removed using 95 % TFA, 2.5 % TIS (triisopropylsilane) and 2.5 % H₂O, followed by precipitation with cold diethyl ether. The products were purified by HPLC on a Phenomenex Luna C18(2) column (10 µm, 250 mm x 21.2 mm) and a dual absorbance detector, at a flow rate of 20 ml/min.

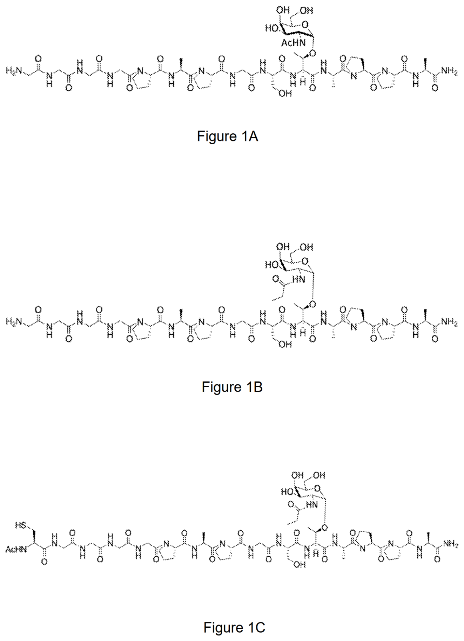

Following this methodology with the amino acids adequately protected, various glycopeptides were synthesised and purified by semi-preparative HPLC. They include the natural sequence (glycopeptide 21 shown in Figure 1A), a modified glycopeptide (24) (shown in Figure 1B), and the same glycopeptide with a cysteine added for binding to the nanoparticles (glycopeptide 33) (shown in Figure 1C).

These are the specific characteristics of these glycopeptides:
Glycopeptide **21** (GGGGPAPGST(GaINAc)APPA):
Following the MW-SPPS methodology with the amino acids adequately protected, the glycopeptide 21 was obtained and purified by semi-preparative HPLC.

Semi-preparative HPLC: Rt=12.7 min (Phenomenex Luna C18 (2), 10 µm, 21.2x250 mm, Grad: acetonitrile/water+ 0.1 % TFA (5:95) → (12.5:87.5), 15 min, 20 ml/min, λ= 212nm).

Analytical HPLC: Rt=15.8 min (Phenomenex Luna C18 (2), 5 µm, 4.6x250 mm, Grad: acetonitrile/water+ 0.1 % TFA (5:95) → (15:85), 20 min, 1 ml/min, λ= 212nm).

HRMS (ESI) m/z: [M+H]+ Calculated for C₅₄H₈₇N₁₆O₂₁: 1295,6226; 1295,6184 found.

Glycopeptide **24** (GGGGPAPGST(GaINCOEt)APPA):
Following the MW-SPPS methodology with the amino acids adequately protected, the glycopeptide 24 was obtained and purified by semi-preparative HPLC.

Semi-preparative HPLC: Rt=14.9 min (Phenomenex Luna C18 (2), 10 µm, 21.2x250 mm, Grad: acetonitrile/water+ 0.1 % TFA (5:95) → (12.5:87.5), 15 min, 20 ml/min, λ= 212nm).

Analytical HPLC: Rt=15.8 min (Phenomenex Luna C18 (2), 5 µm, 4.6x250 mm, Grad: acetonitrile/water+ 0.1 % TFA (5:95) → (20:80), 30 min, 1 ml/min, λ= 212nm).

HRMS (ESI) m/z: [M+H]+ Calculated for C₅₅H₈₉N₁₆O₂₁: 1309,6383; 1309,6327 found.

Glycopeptide 33 (CGGGGPAPGST(GaINCOEt)APPA):
Semi-preparative HPLC: Rt=19, 1 min (Phenomenex Luna C18 (2), 10 µm, 21.2x250 mm, Grad: acetonitrile/water+ 0.1 % TFA (5:95) → (16:84), 22 min, 20 ml/min, λ= 212nm).

Analytical HPLC: Rt=15.8 min (Phenomenex Luna C18 (2), 5 µm, 4.6x250 mm, Grad: acetonitrile/water+ 0.1 % TFA (5:95) → (20:80), 30 min, 1 ml/min, λ= 212nm).

HRMS (ESI) m/z: [M+H]⁺ Calculated for C₆₀H₉₆N₁₇O₂₃S: 1454,6580; 1454,6514 found.

### 2) Antigen-antibody binding studies by Surface Plasmon Resonance (SPR)

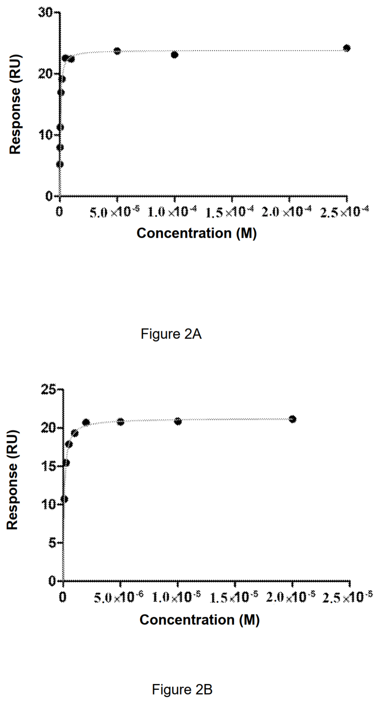

Surface plasmon resonance (SPR) experiments were performed with a Biacore X-100 apparatus (Biacore, GE) in HBS-EP buffer solution at 7.5 pH (10 mM Hepes, 150 mM NaCl, 3 mM EDTA, with 2 % DMSO and 0.05 % Tween X100) at 25 °C. This is the running buffer. The 5E5 antibody (from Abcam) was immobilised on a CM5 sensor chip (Biacore, GE) following the standard amine coupling method.³⁰ Briefly, the carboxymethyl dextran surface of flow cell 2 was activated by a 7 min injection of a 1:1 ratio of a 0.4 M aqueous solution of 1-ethyl-3-(3-dimethylaminopropyl) carbodiimide (EDC) and 0.1 M sulfo-N-hydroxysuccinimide. As such, the antibody was bound to the surface using various dilutions in 10 mM sodium acetate, 4.0. pH during a 7 min injection. Active esters that did not react on the surface were inactivated by a 7 min injection of 0.1 M ethanolamine-HCl in water (8.0 pH). Immobilisation levels were found to be around 5500 resonance units (RU). The flow cell 1 was used as a reference and treated as the flow cell 2 (amine coupling method) without protein. Before use, 50 mM stock solutions of peptide ligand were diluted to the final concentration in the running buffer. Therefore, a series of different compounds were injected into the sensor chip at a flow rate of 30 µl/min for a period of 1 min followed by a dissociation period of 1 min. No regeneration was required. Figures 2A and 2B show the response-concentration curves obtained for the binding of glycopeptide **21** and **24,** respectively, towards the 5E5 (anti-MUC1) antibody at 20 °C in SPR experiments.

The sensogram data were obtained in duplicate and processed with the Biae Evaluation X100 software (Biacore, GE). Experimental data from the affinity measurements were fitted to a specific two-site binding model using Prism software. The resulting dissociation constants (Kd) are shown in Table 1.

**Table 1. Thermodynamic binding parameters obtained by SPR for 5E5 with various glycopeptides at 20 °C and 7.5 pH.**

| Compound | Peptide | K_{D}(µM) | Rmax (RU) | Chi² (RU²) |
|---|---|---|---|---|
| 21 | GGGGPAPGST(GalNAc)APPA | 0.54 | 23 | 0.5 |
| 24 | GGGGPAPGST(GalNCOEt)APPA | 0.083 | 23 | 0.04 |

Therefore, the non-natural glycopeptide **(24)** has an affinity 6.5 times higher (= lower dissociation) than the natural peptide **(21).**

### 3) Nanoparticle synthesis and peptide conjugation

All glassware used for nanoparticle preparation was cleaned with aqua regia (HCl (37 %)/HNO₃ (65 %) 3:1). Ultrapure deionised water (Millipore Elix^{®} 35 water purification system, 18.2 MΩ cm) was used for the preparation of all aqueous solutions. All solutions used for nanoparticle preparation were filtered through a 0.2 µm membrane filter (cellulose acetate, Whatman^{®}). Gold nanoparticles (AuNPs) were characterised by dynamic light scattering (DLS) (Zetasizer, Malvern Instruments or Litesizer500, Antón Paar). The mean diameter of AuNPs was measured using TEM (JEOL JEM-1011 transmission electron microscope operating at an accelerating voltage of 100 kV). UV/Vis measurements were carried out using a TECAN Infinite M200 Pro plate reader. Gold concentration was determined by inductively coupled plasma optical emission spectrometry (Agilent 720 ICP-OES) or inductively coupled plasma mass spectrometry (iCAP^{™} RQ ICP-MS -Thermo Scientific).

The AuNPs coated with citrate, with a diameter of 13.0±1.0 nm, were synthesised according to a published method.³¹ The AuNPs were passivated by forming self-assembled monolayers of alkyl-PEG600 thiols on their surface.³² A mixture of carboxy- and amino-terminated PEG600 thiols (I and II) was used with the mole fraction of the amino-terminated derivative being χ_{NH2} =0.06. Stock solutions of thiols in EtOH (5 mM) were prepared immediately before use and directly used.

The final concentrations in the passivation reaction were: 80-90 nM AuNP, 25 mM NaHCO₃, 1 mM thiols (total) and therefore the reaction contained 20 % v/v EtOH. Stirring was continued at room temperature for 96 h after which the PEG-AuNPs were purified by ultrafiltration on Amicon Ultra-15 filters (1x15 ml 25 mM NaHCO₃, 1x15 ml 50 mM EtOH/NaHCO₃ 2:8 v/v and 2x15 ml 50 mM NaHCO₃ buffer solution). Purified PEG-AuNPs were dispersed in H₂O at a final concentration between 200 and 400 nM.

The peptides described above were coupled to PEG-AuNPs according to a previously reported method.³³ Briefly, SM(PEG)₂ derivative was added at a final concentration of 5 mM to a sample of AuNPs functionalised with amino groups in 30 mM phosphate buffer at 8.2 pH. The concentration of AuNP in the reaction mixture can be as high as 400 nM. The reaction mixture was stirred for 4 h at 1 °C. The thus functionalised AuNPs were purified by ultrafiltration (2x4 ml EtOH/20 mM phosphate buffer at 8.2 pH 2:8 and 2x4 ml H₂O) by performing all steps on ice, in a centrifuge which is cooled to 4 °C, and with cold wash solutions. The purified functionalised AuNP particles were dispersed in water. In parallel, 200 µl of a 1 mM dilution in water of the peptide was incubated with 10 mM tris(2-carboxyethyl)phosphine hydrochloride (TCEP) for 2 h at 0 °C to completely reduce the formation of glycopeptide-derivative disulphides.

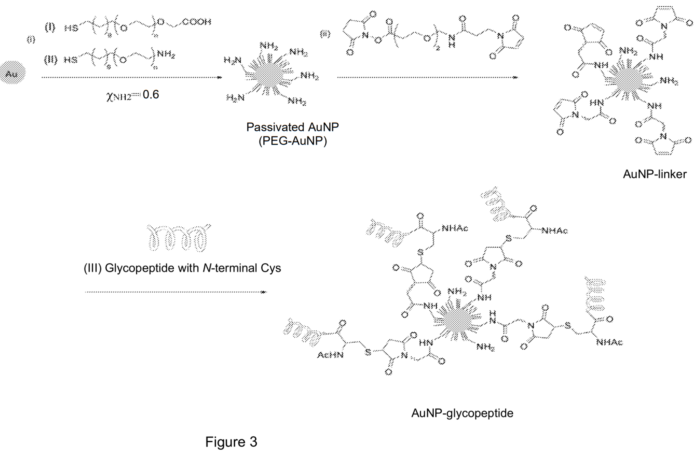

For the coupling reaction, the required amount of freshly TCEP-treated peptide was added to a freshly prepared cold solution of functionalised AuNPs and stirred overnight at 4 °C, at 500 rpm. The typical reaction volume was 500 µl and the final concentrations in the reaction mixture were: Functionalised AuNPs ~100 nM, 15 mM phosphate buffer at 7.0 pH, 50 µM peptide. Excess uncoupled peptide was removed by means of centrifuging, removing the supernatant, and resuspending in 1 ml buffer (three cycles), 20800 xg, 45 min, 18 °C), resuspending for the first time in 1 ml of 25 mM NaHCO₃, then twice in 10 mM NaHCO₃. Lastly, the AuNPs coupled to purified peptides were treated with 350 µl of 10 mM NaHCO₃ to reach a final concentration of about 100 nM, which was stored protected from light at +4 °C. The method is schematically depicted in Figure 3.

The hydrodynamic radius of the AuNPs was determined by DLS. The AuNPs were suspended in 10 mM NaHCO₃. The ζ potentials were measured in the same buffer. The values are reported in Table 2.

**Table 2. Physicochemical characterisation of the AuNPs used in this work.**

| | DLS^{a} | | Potential ζ^{a} |
|---|---|---|---|
| AuNP | Ø(nm)^{b} | PDI | (mV) |
| AuNP-glycopeptide 33 | 28±1 | 0.127 | -33±4 |

| | | | |
|---|---|---|---|
| ^{a} Measurements in 10 mM NaHCO₃.^{b} Derived from intensity distributions. | | | |

### 4) Dot blot assay

The AuNPs were placed in triplicate with a capillary on pre-moistened nitrocellulose membranes (AuNP, 16 nM) and allowed to dry at room temperature (ta) for at least 2 h.

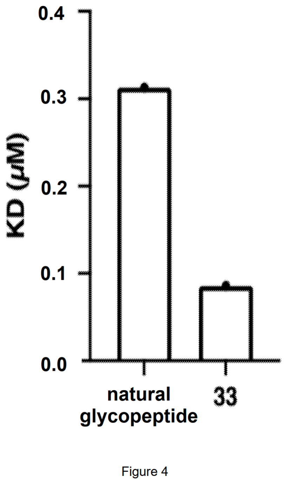

For the affinity tests (results shown in Figure 4), each membrane was blocked with 5 % skimmed milk in tris-buffered saline (TBS) for 1 h, was washed in TBS with 0.1 % Tween 20 (3x10 min) and incubated overnight at 4 °C with the mouse monoclonal anti-MUC1 5E5 antibody provided by Dr Clausen (University of Copenhagen) (1 µg/ml at 0,000122 µg/ml in 5 % milk in TBS). After washing with TBS containing 0.1 % Tween 20 (3x10 min), the membranes were incubated with goat-derived Dylight 800 mouse anti-IgG (H+L) secondary antibody (039610-145-121, Tebu-bio), diluted 1:2500 in TBS for 1 hour at room temperature.

Lastly, membranes were washed in TBS with 0.1 % Tween 20 (3x10 min) and TBS (2x10 min) and visualised using a LI-COR Biosciences Odyssey Infrared Imaging System. The intensity of the marks was quantified by densitometry with ImageJ, subtracting the bottom.

### 5) Serum analysis of pancreatic cancer patients and healthy volunteers

The study was designed as a retrospective, observational, and longitudinal clinical study. All data were anonymised. Personal and clinical data collected for the study in accordance with the Spanish Data Protection Act (Organic Law 3/2018 of 5 December for Personal Data Protection). The study adheres to all the principles of the Declaration of Helsinki and was approved by the local review committee (Clinical Research Ethics Committee of La Rioja, Ref. CEICLAR P.I. 260). Blood was collected from all subjects who signed the informed consent form and serum was drawn, which was subjected to an immunoreactivity assay against MUC1 antigen using the dot blot technique.

A group of 20 pancreatic cancer patients participated in the study (65 % male, 65.2±9.0 years) and 20 healthy volunteers matched by age and sex (65% male, 63.4±7.5 years) (Hospital San Pedro, Logroño, Spain). No significant differences were found between the groups with respect to both age and sex ratios (Table 3).

**Table 3. Age and sex distribution of cancer patients and healthy volunteers in the study. Statistical tests used: Chi-square (*) or t-test (^{†}).**

| | Healthy volunteers (n=20) | Cancer patients (n=20) | *value* of p |
|---|---|---|---|
| Sex (men, %) | 13 (65%) | 13 (65%) | 1.000* |
| Age (years) | 63.4±7.5 years | 65.2±9.0 | 0.507^{†} |

For serum analysis, each membrane was functionalised with nanoparticles as described in section 4, was blocked with 5 % BSA in TBS for 1 h, was washed in TBS with 0.1 % Tween 20 (3x10 min) and incubated overnight at 4 °C with patient serum (1:100 dilution in TBS with 1 % BSA). After washing with TBS containing 0.1 % Tween 20 (3x10 min), the membranes were incubated with rabbit anti-human IgG (H+L) secondary antibody Dylight 800 (039609-445-002, Tebu-bio), diluted 1:5000 in TBS for 1 h at room temperature. Lastly, membranes were washed in TBS with 0.1 % Tween 20 (3x10 min) and TBS (2x10 min) and visualised using a LI-COR Biosciences Odyssey Infrared Imaging System. The intensity of the marks was quantified by densitometry with ImageJ, subtracting the bottom.

Each serum was analysed in triplicate, and the assay was performed in triplicate (three different membranes for each serum). The first normalisation was performed on cases and controls separately by using the inter-array function of the Limma library (R main library) within each acquisition. This was done to avoid the loss of the effect of the disease. Then, the intensities of all acquisitions were normalised to the total average of all procurements. This is necessary to eliminate the intensity bias associated with the different acquisitions made with the fluorescence scanner. In order to carry out a statistical data analysis, the triplicates of the samples were merged, averaging the intensities for each peptide-AuNP tested.

For statistical analysis, the normality of the data distribution (Shapiro-Wilk test) and homoscedasticity (Levene's test) were verified. When the data sets were normally distributed with equal variances, t-test was used. Welch's test was applied in case of normal data and heteroscedasticity. The non-parametric Mann Whitney U test was used for non-normal data. The significance level was set at *p* < 0.05.

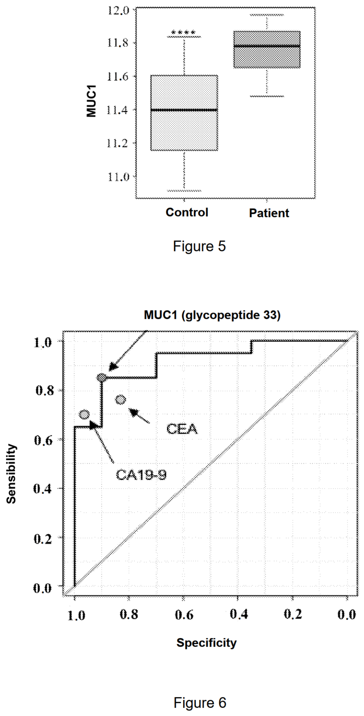

The levels of anti-MUC1 antibodies found in cancer patients (11.76±0.14 arbitrary fluorescence units, AFU) were statistically significantly higher (p<0.0001) than in healthy volunteers (11.38±0.26 AFU) (Figure 5).

To better understand the potential of our MUC1 immunoreactivity system as a diagnostic tool, a receiver operating characteristic (ROC) curve was constructed. An area under the curve (AUC) of 0.918 (95% IC) was obtained: 0.832-1.000). The optimal threshold was calculated at 11.63 AFU for the maximum sum of sensitivity (85 %) plus specificity (90 %) (Figure 6).

Comparing these values with those of two tumour markers currently in use for pancreatic cancer monitoring, the advantage of our system is quite clear (Figure 6, Table 4).

**Table 4. Sensitivity and specificity values of two tumour markers currently used for pancreatic cancer monitoring and results obtained for our dot blot assay using nanoparticles (glycopeptide 33)**

| | Sensitivity | Specificity | AUC |
|---|---|---|---|
| CEA²³ | 77.0 | 83.2 | approximately 0.8 (unpublished) |
| CA19-9²² | 69.5 | 98.2 | 0.875 |
| NP-glycopeptide **33** | 85.0 | 90.0 | 0.918 |

### References

(1) Taylor-Papadimitriou, J.; Burchell, J.; Miller, D.W. Dalziel, M. Biochim. Biophys. Acta 1999, 1455:301-313.
(2) Kufe, D. W. Nat. Rev Cancer 2009, 9 (12), 874-885.
(3) Levitin, F.; Stern, O.; Weiss, M.; Gil-Henn, C.; Ziv, R.; Prokocimer, Z.; Smorodinsky, N. I.; Rubinstein, D. B.; Wreschner, D. H. J. Biol. Chem. 2005, 280 (39), 33374-33386.
(4) Boshell, M.; Lalani, E.; Pemberton, L.; Burchell, J.; Gendler, S.; Taylor-Papadimitriou, J. Biochem. Biophys. Res. Commun. 1992, 185 (1), 1-8.
(5) Smorodinsky, N.; Weiss, M.; Hartmann, M.; Baruch, A.; Harness, E.; Yaakobovitz, M.; Keydar, I.; Wreschner, D. Biochem. Biophys. Res. Commun. 1996, 228 (1), 115-121.
(6) Yousefi, M.; Dehghani, S.; Nosrati, R.; Zare, H.; Evazalipour, M.; Mosafer, J.; Tehrani, B. S.; Pasdar, A.; Mokhtarzadeh, A.; Ramezani, M. Biosens. Bioelectron. 2019, 130, 1-19.
(7) Moreno, M.; Bontkes, H. J.; Scheper, R. J.; Kenemans, P; Verheijen, R. H. M.; von Mensdorff-Pouilly, S. Cancer Lett. 2007, 257 (1), 47-55.
(8) Frenette, P. S.; Thirlwell, M. P; Trudeau, M.; Thomson, D. M. P; Joseph, L.; Shuster, J. S. Tumor Biol. 1994, 15 (5), 247-254.
(9) Rughetti, A.; Fama, A.; von Mensdorff-Pouilly, S.; Taurino, F.; Rahimi, H.; Ribersani, M.; Natalino, F.; D'Elia, G. M.; Bizzoni, L.; Latagliata, R.; et al Blood 2008, 112 (11), 5237-5237.
(10) Tothill, I. Semin. Cell Dev. Biol. 2009, 20 (1), 55-62.
(11) Meyer, T.; Rustin, G. Br. J. Cancer 2000, 82 (9), 1535-1538.
(12) Basil, C.; Zhao, Y.; Zavaglia, K.; Jin, P; Panelli, M.; Voiculescu, S.; Mandruzzato, S.; Lee, H.; Seliger, B.; Freedman, R.; et al Cancer Res. 2006, 66 (6), 2953-2961.
(13) Bohunicky, B.; Mousa, S. Nanotechnol. Sci. Appl. 2010, 4 (1), 1-10.
(14) Tang, Y.; Wang, L.; Zhang, P; Wei, H.; Gao, R.; Liu, X.; Yu, Y.; Wang, L. Clin. Vaccine Immunol. 2010, 17 (12), 1903-1908.
(15) Gheybi, E.; Amani, J.; Salmanian, A. H.; Mashayekhi, F.; Khodi, S. Tumor Biol. 2014, 35 (11), 11489-11497.
(16) Sung, H.; Ferlay, J.; Siegel, R. L.; Laversanne, M.; Soerjomataram, I.; Jemal, A.; Bray, F. CA. Cancer J. Clin. 2021, 71 (3), 209-249.
(17) Spain: number of deaths from cancer by type 2019 | Statista https://www.statista.com/ statistics/777097/number-of-deaths-from-cancer-in-spain-by-type-of-cancer/ (consulted on 3 November 2021).
(18) Bose, M.; Mukherjee, P. Vaccines 2020, 8 (4), 1-21.
(19) Curry, J. M.; Thompson, K. J.; Rao, S. G.; Besmer, D. M.; Murphy, A. M.; Grdzellshvlll, V. Z.; Ahrens, W. A.; McKillop, I. H.; Sindram, D.; lannlttl, D. A.; et al J. Surg. Oncol. 2013, 107 (7), 713-722.
(20) Tinder, T.; Subramani, D.; Basu, G.; Bradley, J.; Schettlnl, J.; Million, A.; Skaar, T.; Mukherjee, P. J. Immunol. 2008, 181 (5), 3116-3125.
(21) Hamanakai, Y.; Suehiro, Y.; Fukui, M.; Shlklchl, K.; Imai, K.; Hinoda, Y. Int. J. Cancer 2003, 103 (1), 97-100.
(22) Murakami, M.; Nagal, Y.; Tenjin, A.; Tanaka, Y. Endocr. J. 2018, 65 (6), 639-643.
(23) Pasanen, P. A.; Eskelinen, M.; Partanen, K.; Pikkarainen, P; Penttlla, I.; Alhava, E. Br. J. Cancer 1993, 67 (4), 852-855.
(24) Martínez-Sáez, N.; Peregrina, J. M.; Corzana, F. Chem. Soc. Rev. 2017, 46 (23), 7154-7175.
(25) Compañón, I.; Guerreiro, A.; Mangini, V.; Castro-López, J.; Escudero-Casao, M.; Avenoza, A.; Busto, J. H.; Castillo, S.; Asensio, J. L.; Jiménez-Osés, G.; et al J. Am. Chem. Soc. 2019, 141, 4063-4072.
(26) Macías-León, J.; Bermejo, I. A.; Asín, A.; García-García, A.; Compañón, I.; Jiménez-Moreno, E.; Coelho, H.; Manglnl, V.; Albuquerque, I. S.; Marcelo, F.; Asenslo, J.L.; Bernardes, G. J.L.; Joshi, H. J.; Fiammengo, R.; Blixt, O.; Hurtado-Guerrero, R.; Corzana, F. *Chem. Commun.* 2020, *56,* 15137-15140.
(27) Y. Zhang, M. Li, X. Gao, Y. Chen, T. Llu, Journal of Hematology & Oncology 2019, 12, 137.
(28) S. Jain, K. Raza, AK Agrawal, A. Vaidya, in Nanotechnology Applications for Cancer Chemotherapy (Eds.: S. Jaln, K Raza, AK Agrawal, A. Valdya), Elsevier, 2021, pp. 631-663.
(29) Bermejo, I.A.; Usabiaga, I.; Compañón, I.; et al *J. Am. Chem. Soc.* 2018, *140,* 9952-9960.
(30) Johnsson, B.; Löfås, S.; Lindquist, G. Anal. Biochem. 1991, 198 (2), 268-277.
(31) Grabar, K. C.; Freeman, R. G.; Hommer, M. B.; Natan, M. J. Anal. Chem. 2002, 67 (4), 735-743.
(32) Maus, L.; Dick, O.; Bading, H.; Spatz, J. R; Fiammengo, R. ACS Nano 2010, 4 (11), 6617-6628.
(33) Cai, H.; Degliangeli, F.; Palitzsch, B.; Gerlitzki, B.; Kunz, H.; Schmitt, E.; Fiammengo, R.; Westerlind, U. Bioorg. Med. Chem. 2016, 24 (5), 1132-1135.

## Claims

1. Synthetic non-natural glycopeptides, as represented by [GGGGPGPAPGST(GaINCOEt)APPA] and/or [CGGGGPAPGST(GaINCOEt)APPA] and/or their derivative synthetic glycopeptides for use as cancer biomarker detectors in the detection of early stage tumours.

2. A biomarker binding platform comprising a nanoparticle and a covalently bound synthetic non-natural glycopeptide antigen, as represented by [CGGGGPAPGST(GaINCOEt)APPA] and/or its derivative glycopeptide antigens for use as cancer biomarker detectors in the detection of early stage tumours.

3. The binding platform according to claim 2 wherein the nanoparticle comprises a gold nanoparticle.

4. The binding platform according to claim 2, wherein the nanoparticle comprises micelles, dendrimers, liposomes, hybrid and compact polymeric nanoparticles, fullerenes, carbon dots, quantum dots, silica and metal nanoparticles.

5. The binding platform according to claims 2 to 4, wherein the non-natural synthetic glycopeptide is [CGGGGPAPGST(GaINCOEt)APPA].

6. A kit for performing an assay for detecting and quantifying binding between anti-MUC1 antibodies produced by the patient from a sample and a binding platform according to any of claims 1 to 5.

7. The kit according to claim 6, wherein the assay is selected from the group including dot blot, Western Blot, ELISA, SPR and liquid phase infrared spectroscopy.
